# EUROPEAN PATENT APPLICATION

(11) **EP 4 497 429 A1**
(43) Date of publication of application: **29.01.2025**
(21) Application number: 23183844.2
(22) Date of filing: 06.07.2023
(51) Int. Cl.: A61K 8/33, A61K 8/34, A61K 8/92, A61Q 11/00, A61Q 11/02, A61K 8/31, A61K 9/00, A61K 8/39

(54) **CARE SOLUTION, IN PARTICULAR FOR USE WITH 3D-PRINTED POLYMER DENTAL APPLIANCES**

(71) Applicant: Bottmedical AG, 4057 Basel (CH)
(72) Inventor: TÖPPER, Tino, 79115 Freiburg (DE)
(74) Representative: Mertzlufft-Paufler, Cornelius

(57) **Abstract**

A novel composition is proposed for a water-based care solution that may be employed for increasing the hygiene of intraoral appliances such as dental splints. By immersing such an appliance into said care solution, different active agents contained in the care solution can be rapidly loaded at sufficiently high amounts into the device, in particular if the device shows a highly porous surface due to an underlying 3D-printing fabrication process. Accordingly, once reloaded, the dental appliance can release the different agents into the oral cavity to achieve an antimicrobial effect inside the oral cavity and the agents remaining in the pores of the device can prevent microorganisms from growing on or inside the appliance.

## Description

The present disclosure relates to the field of intraoral/ dental appliances worn by a user inside his oral cavity, typically directly on his teeth of the lower or upper jaw. More particularly, the invention relates to an aqueous solution with antimicrobial properties that is tailor-made for treating such dental appliances and for increasing their hygiene. However, the solution may be used in other applications, such as mouthwash or for cleaning surfaces in general. As another effect, such an aqueous solution can have a direct (if applied directly to the mouth) or indirect (if loaded into the appliance) positive influence on the balance of the oral microbiome of the patient, the microbiome being the natural and healthy composition of bacteria in the oral cavity.

Oral health is often taken for granted. However, oral inflammations remain common and may lead to other illnesses, including heart disease, diabetes, and neurological disorders like Alzheimer's. Good oral health is associated with good overall health and could be linked to a reduced risk of severe virus infections.

Intraoral orthodontic devices are mainly used in orthodontic therapy for realigning teeth. In this specific application case, the devices are typically designed as a dental splint, which is applied to teeth over several hours. The dental splint exerts forces on the patient's individual teeth, which results in a realignment of the teeth in the directions of desired teeth positions. Typically, a number of dental splints, which vary slightly from each other in shape, are worn by the patient consecutively to realign the teeth stepwise. Further known applications of such intraoral devices, in particular dental splints, are bleaching of teeth using bleaching pastes which are applied to the teeth by the device, or the use of such devices for treating snoring overnight. Intraoral appliances are also used for avoiding clenching and grinding and for aligning the upper and lower jaw to each other.

In all of these applications, it is crucial that the hygiene of dental appliances and the oral hygiene of the patient is maintained as a preventive measure to avoid and/or treat serious illnesses of the teeth and gums of the user. In particular, providing a high safety against infections caused by microorganisms growing on or inside the dental appliance is of central importance for a high safety of use. This becomes particularly important for patients who already have a low overall state of health or who are already suffering from previous illnesses inside the oral cavity. As invisible braces applied as clear aligners must be worn extensively during the night and even through the day with only minimal removal periods for meals and cleaning, decreasing the risk of biofilm formation and subsequent damage to tooth substance and soft tissue is undoubtedly a valuable preventive strategy for these devices in particular.

It is well-known that dental appliances can also be designed and employed as functional devices, which can deliver active agents to the user's oral cavity, thereby providing an antimicrobial effect, for example. In such a case, the dental appliance, in particular an outer layer of the dental appliance, acts as a reservoir (or offers several reservoirs) that stores the active agents and releases them into the cavity once the user is wearing the appliance in his oral cavity. Moreover, the presence of the agents in the intraoral appliance can also effectively prevent the growth of microorganisms on or inside the appliance. Most common are designs, in which the polymer material itself, which forms the dental appliance, acts as the reservoir for the agent.

The present invention also builds on knowledge from prior art, in particular as described in WO 2022 033985 A1: This publication discusses the effectiveness of certain agents for suppressing the growth of bacteria such as streptococcus mutans and streptococcus mitis on a particular material system comprising cellulose. Based on experimental measurements of the lag phase (postponement in time of the growth of the bacteria, measured in hours) and growth rate (increase in number of bacteria/time, measured in J/h), limonene and cinnamaldehyde were found to be particularly efficient agents against streptococcus mutans, while the agent methyl salicylate was found to be highly effective against streptococcus mitis. In this publication, it is also discussed that the polarities of the agents seem to impact their respective interaction and thereby the reload speed (the speed at which the respective agent can be loaded into a dental appliance), while the ratio of the surfactant was also found to be important. In particular, it was found that cinnamaldehyde offers superior reload speed in cellulose-based cap layers as compared to limonene and methyl salicylate.

For achieving the best protection against the above-mentioned bacteria, WO 2022 033985 A1 proposes doublets composed of two different essential oil components (EOCs) only, namely limonene or cinnamaldehyde on the one hand and methyl salicylate or trans-anethole on the other hand. These doublets are to be used as antimicrobial agents to be loaded into a dental appliance by using a reload-liquid (containing thus doublets comprising two of those agents), into which the appliance is to be immersed prior to use (for loading the agents into the appliance).

WO 2022 033985 A1 also states that using a surfactant such as Polyglyceryl-4 Laurate in the care solution is important for achieving a high load concentration of the agent per surface area (because WO 2022 033985 A1 assumes that the ratio of surfactant to agent defines the speed of loading). WO 2022 033985 A1 discusses suitable ratios of a concentration of a surfactant cₛ in the reload-liquid and a concentration cₐ of one or several agents in the reload-liquid and proposes to employ ratios of cₐ/cₛ above 0.01 or even above 0.1. This technical teaching thus recommends an upper limit for the concentration of the surfactant to values of: cₛ < 10 cₐ. At the same time, WO 2022 033985 A1 states the assumption that the higher the concentration of the surfactant, the faster the re-loading of the agent.

As in many other fields, direct 3D-printing techniques are currently revolutionizing the market of dental appliances because this fabrication approach offers a way for cheap mass-production of patient-specific devices which are tailored in shape to the needs of the individual patient/user using digital technology.

Dental appliances directly 3D-printed from polymer materials, such as dental splints can, for example, be fabricated based on a crosslinking process produced with UV light. Nylon, as another possible material, can be 3D-printed as a powder using a laser printer to form a dental splint. In the latter case, the connection of the powder particles is based on the action of heat (provided selectively by IR laser radiation) to form 3D molded dental splints. A characteristic feature of such 3D-printed dental splints is that they are generally much more porous due to the underlying thermoplastic or UV-based crosslinking process than comparable dental splints produced by classical thermoforming of thermoplastic foils. Accordingly, such devices are much more prone to rapidly developing a hazardous biofilm of growing bacteria and other microorganisms such as fungi.

Based on this technical background, it is an object of the present invention to provide a recipe for an aqueous solution that is useful for increasing the safety in use of dental appliances. In particular, it is an object of the invention to provide an optimized composition of a care solution allowing rapid reloading of a specific combination of natural agents into the outer layers of 3D printed dental appliances, which are highly effective in providing direct antimicrobial protection and/or an indirect influence on the composition of bacteria present in the oral cavity. Another objective is to optimize the reload capacity for different agents contained in the solution; for example, one goal may be to maximize the total amount of the specific combination of agents that can be loaded into a given intraoral appliance.

In accordance with the present invention, an aqueous solution is provided according to claim 1 and/or according to claim 4, which solves the aforementioned problem and may be used as a care solution for treating oral appliances to be worn in the oral cavity. Due to the cleaning and antimicrobial effect provided by the solution, it can effectively inhibit the formation of hazardous biofilms on the dental appliance and thereby increase the safety of use of such devices. And due to the release of the active agents from the appliance, cariogenic biofilm formation can be effectively inhibited inside the oral cavity when a user is wearing such a loaded dental appliance in his mouth.

Moreover, this solution can also be used as a mouth wash, and generally for cleaning and/or disinfecting surfaces, in particular surfaces with significant porosity.

Polymers that can be efficiently cleaned with the solution are, for example, cellulose acetate, cellulose acetate butyrate, PLA, chitosan or polyester, co-polyester, polycarbonate, thermoplastic polyurethane, polypropylene (PP), polyethylene (PE), polypropylene and polyethylene copolymers, acrylic, in particular PMMA, cyclic block copolymers, polyetheretherketone, polyamide, polyethylene terephthalate, polybutylene terephthalate, polyetherimide, polyethersulfone, polytrimethylene terephthalate or a combination thereof (e.g., a blend of at least two of the listed hard polymeric materials). For such polymers, the efficiency of the achievable deep cleaning, which is crucial for reliable antimicrobial protection, depends on the polarity of side groups, the absorption rate of the polymer, the functionalization of the polymer surface, and on the porosity of the polymer network (typically defined by the molecular weight and degree of branching of the polymer chains).

Moreover, the solution may be used as a reload solution for loading/reloading (in particular porous) dental appliances (i.e., for equipping such devices with several different active molecules to be later released from the dental appliance into the oral cavity). For example, when used with a cellulose-based thermoformable material as a dental appliance, the appliance loaded with the solution is well tolerated by human gingival fibroblasts, as cytotoxicity tests have shown. In addition, the loaded material is stable in its mechanical properties, thus offering a reliable transmission of forces as required for orthodontic treatments.

The motivation/insight behind the invention is that independent of the respective reload efficacy of each agent/essential oil component, a combination of different agents/different essential oil components can produce synergistic effects (most importantly against oral streptococci), in particular, if the different agents show different polar groups that adhere/interact in different ways with bacteria, e.g., by adhering to cells or by penetrating the membrane of a microorganism or by rendering such a membrane more permeable. As a result, multiple different species of bacteria can be combatted with the solution in a synergistic way.

For example, cinnamaldehyde has a polar group with high polarity, while limonene has basically no such comparable polar group. These differences in the chemistry of the single agents are beneficial for attacking different microorganisms (for example, limonene has been shown to inhibit acid resistance of certain bacteria) but also pose challenges in terms of achieving high reloading for all agents contained in the solution, in particular when loading porous polymer materials.

The invention has also realized, on the other hand, that the high porosity of 3d-printed materials also offers some advantages: by designing/choosing a suitable porosity of the dental appliance, it is possible to tailor the reload capacity and reload speed for molecules contained in an aqueous solution, for example as described herein. The higher the porosity, the higher the reload speed and the release of functional molecules into the dental appliance.

In particular, the invention proposes an aqueous solution with antimicrobial properties as introduced at the beginning, which, in addition, is characterized in that the solution comprises the following ingredients: (at least or exclusively) a triplet of three different oleophilic antimicrobial essential oil components (which function as active agents) and a surfactant.

An essential oil component (EOC), such as cinnamaldehyde, may be understood here as a substance comprised in a natural essential oil (e.g. cinnamon oil). Essential oils (EOs) are liquid and volatile substances extracted from plants. Many of the substances (EOCs) comprised in an EO interact with the cell membrane of bacteria due to their hydrophobic/oleophilic nature, making the cell more permeable and potentially leading to cell death. EOCs/EOs have been proven to have antifungal, antibacterial, antiviral, and insecticidal properties. In addition, EOCs/EOs show a low-level of antimicrobial resistance and a broad spectrum of antimicrobial activity. Among the EOCs, cinnamon has an important role because of its antioxidant, anti-inflammatory, antidiabetic, antimicrobial, antitumoral, and lipid-lowering properties; it is also effective against caries-causing bacteria. The main bioactive molecule in cinnamon oil is cinnamaldehyde, which has been recognized as safe and nontoxic by the FDA.

EOCs can also inhibit the growth of pathogens responsible for gingivitis and periodontitis, such as Porphyromonas gingivalis, and can limit the inflammation caused by Aggregatibacter actinomycetemcomitans. In contrast to high-concentrated mouthwash or other liquid disinfectants like chlorohexidine, the continued and targeted release of EOCs from a dental appliance loaded with an aqueous solution, according to the invention, enables that body-friendly and non-cytotoxic doses of these agents, still achieve significant antibiofilm efficacy. In addition, appliances loaded with such a solution can remain mechanically stable such that they can be used in the intended way.

The surfactant may be preferably in the form of a polyglyceryl-based emulsifier, most preferably PG-4 (which is a natural water-in-oil emulsifier based on sunflower derived polyglyceryl-4 ester). We note that surfactants can be described as "surface active substances". Typically, surfactants are found in the form of compounds that are amphiphilic, meaning they have a polar or hydrophilic, i.e., water-soluble, part and a nonpolar, i.e., hydrophobic or lipophilic, part. Therefore, surfactants can decrease the surface tension of an aqueous solution. A surfactant can generally act as a detergent, a wetting agent, and as an emulsifier. An emulsifier is a substance that stabilizes an emulsion by reducing the oil-water interfacial tension. Emulsifiers are thus part of the broader group of surfactants. Emulsifiers that are more soluble in water, and conversely less soluble in oil, will generally form oil-in-water emulsions. Emulsifiers are not to be confused with solubilizers, which are compounds that increase the solubility of a substance in a solvent, either by changing the properties of the solvent or by micelle formation.

The invention thus proposes a combination of at least three different active agents, because it has been found that triplets offer superior suppression of the growth of microorganisms as compared to doublets (even if the single amount of agent that can be loaded is lower as compared to a doublet). This new finding is particularly true when applying the solution to 3D-printed polymer materials / 3D-printed intraoral appliances, where loading of several different agents at sufficiently high amounts can, however, be challenging. Efficient and rapid loading for such materials is possible for the specific combination of methyl salicylate, cinnamaldehyde, and limonene, which also offers excellent antimicrobial protection.

Most preferably, said triplet can therefore comprise methyl salicylate (C8H8O3), cinnamaldehyde (C9H8O), and limonene (C10H16). This particular triplet offers superior efficiency in terms of protection against various microorganisms, because these substances can be loaded together and show differences in their respective polarity (methyl salicylate offers a Carboxy-group (-COOH); cinnamaldehyde has no such group but offers a polar aldehyde-group; limonene, on the other hand, has neither a COOH- nor an aldehyde-group but offers a C=C-binding), resulting in different antimicrobial effects which can synergistically support each other.

We also note that the different hydrophobicity / lipophilicity is responsible for the different antimicrobial effects: Limonene is most hydrophobic, as it consists mainly of nonpolar carbon-hydrogen bonds. Cinnamaldehyde and eucalyptol are also hydrophobic but have slightly more polarity. Methyl salicylate contains a polar carboxy group (-COOH), which leads to some solubility in water, accordingly it is less hydrophobic than the previously mentioned substances. Trans-anethole, for example, contains a methoxy group (-OCH3), which has a polarized C-O bond. This leads to some solubility in water, indicating that Trans-anethole is somewhat less hydrophobic compared to the other substances. In summary, limonene is probably the most hydrophobic substance in the above list, followed by cinnamaldehyde, eucalyptol, methyl salicylate and trans-anethole.

Other similarly suitable EOCs that may be used instead of limonene or in addition to limonene as an ingredient of the solution are eucalyptol, trans-anethole and/or carvacrol and/or linalool and/or linalyl-acetate and/or eugenol and/or thymol.

According to another embodiment, the solution can comprise a quadruplet of different oleophilic antimicrobial agents, namely methyl salicylate, cinnamaldehyde, limonene, and eucalyptol.

According to yet another embodiment, the solution can comprise a quintet of different oleophilic antimicrobial agents, namely methyl salicylate, cinnamaldehyde, limonene, eucalyptol and trans-anethole. It has been found experimentally that the combination of these EOCs can be effectively loaded together into various polymer materials from which dental appliances can be fabricated.

As mentioned, the agents can be contained in the solution in the form of emulsified essential oil components. We also note that all agents mentioned above can be obtained from natural sources.

Another way of describing a possible aqueous solution according to the invention which solves the afore mentioned problem is that the solution (which may be used as described previously in various ways) comprises a combination of at least two different antimicrobial agents in the form of two different oleophilic essential oil components (EOCs), which show different polarities, because only one of the two different EOCs has a side chain featuring a polar end group such as a COH-group (as found in aldehydes), a group featuring a carbon-oxygen double bond, an ether group, a carboxy group, a hydroxyl group or a methoxy group. In other words, the other EOC does not feature said end group. Accordingly, the other EOC contained in the solution is less polar than the EOC featuring said polar end group. This is detailed in claim 4. It should be noted here that the EOCs can show a global polarity that is rather low, thus rendering the complete molecule oleophilic. Due to the polar group in one of the side or end groups, however, the respective EOC can show a substantial polarity locally on a molecular level, and this high local polarity can be employed for attacking the membranes of microorganisms, resulting in an efficient antimicrobial effect.

As at least one polar end group is therefore present only in one of the two different EOCs, the EOCs differ in their respective polarities on a local molecular level. As a result, each EOC will interact differently with the membranes of various microorganisms, resulting in a synergistic effect in terms of antimicrobial protection. It is noted here that such a solution can also have features as detailed in claim 1 and/or as described above.

In terms of difference of polarity, the largest difference can be achieved if said polar end group, which is only present in one of the two different EOCs, is a Carboxy-group (-COOH), which is therefore preferable.

Most preferably, the solution can comprise a triplet of three different EOCs, wherein each of said three EOCs differs in its respective polarity from the two other (remaining) EOCs, respectively, due to the presence or non-presence of at least one polar end group or side group (as detailed above for the case to two different EOCs). Such a specific choice of the EOCs present in the solution will further increase the efficacy of the antimicrobial protection provided by the solution.

The aqueous solution, in particular as detailed in the beginning, comprising two or several different antimicrobial agents can be further enhanced if the solution comprises at least one prebiotic, e.g., a nitrate (NO3)-based substance. Prebiotics are compounds in food that foster the growth or activity of beneficial microorganisms, such as bacteria supporting the eubiosis in the oral cavity. Nitrate-based substances are characterized in that they can be reduced/metabolized by bacteria present in the oral cavity to nitric oxide (NO), the latter playing a key role in the physiology of a variety of central body functions. Applying such a nitrate-based substance into the oral cavity along with said mentioned antimicrobial agents results in a localized reduction of the substance by oral bacteria, e.g., by gram-negative bacteria, into nitric oxide. In other words, such a substance can activate a nitrate-reducing metabolism in microorganisms present in the oral cavity as an indirect antimicrobial action mode. This approach can induce nitrosative stress and finally results (indirectly) in reduced growth rates of related bacteria at inflamed gums or in periodontal pockets, as has been proven in recent medical studies.

Furthermore, in addition, or as an alternative to using at least one nitrate (NO3)-based substance in the solution, the invention proposes that the care solution comprises at least one ph-lowering agent, preferably at least one acidic substance for further enhancing antimicrobial protection. Such a substance can be used to lower the ph-value of the saliva when applied to the oral cavity, either directly as a mouthwash or indirectly when released from a dental appliance, as described previously. Importantly in the context of this invention, lowering the ph-value in a patient's mouth is beneficial for increasing the antimicrobial efficiency of the antimicrobial agents contained in the solution. For example, a lower ph-value can ease the penetration of the agents into microbial biofilms present in the oral cavity. The ph-lowering agent can thus increase the antibacterial efficacy of the antimicrobials contained in the solution. As a side effect, such ph-lowering agents, in particular when loaded into aforementioned 3D printed dental appliances, induce bleaching effects to the enamel of the teeth onto which the dental splint is applied.

Preferably, at least one organic acid may therefore be comprised in the solution as a ph-lowering acidic substance, for example, at least one of the following substances: naturally derived malic acid, tartaric acid, carboxylic acid, or citric acid.

An aqueous solution according to the invention is ideally suited to be used as a care solution optimized for 3D-printed dental splints. The end customer can charge/load his dental splint with functional molecules, namely said antimicrobial agents, at regular intervals, for example daily. After loading with the care solution, the dental splint can thus serve as an "agent delivery device".

In other words, the dental splint can be used to deliver a doublet, triplet, quadruplet, or quintet of antimicrobial agents to the oral cavity, preferably combined with at least one nitrate-based substance and/or at least one organic acid, as contained in the care solution and as transferred into the 3D-printed body of the intraoral appliance. In addition, treating an oral appliance with the care solution also achieves a certain degree of cleaning of the appliance, thereby preventing the growth of bacteria on the appliance. As an overall result, the user/patient is thus effectively protected from infections caused by the presence of microorganisms on or in the splint/appliance and/or inside his oral cavity.

Finally, in another possible application case, a solution according to the invention can also be directly applied to the oral cavity as a mouthwash or high-viscose liquid or in a gel-like form, for example, for local treatment of inflamed periodontal pockets.

The ratio of the total mass portion of the antimicrobial agents cₐ contained in the solution, and the mass portion of the surfactant cₛ contained in the solution can preferably be in the range of: cₐ/cₛ = [0.03 - 0.3], preferably cₐ/cₛ = [0.05 - 0.15].

In line with the findings published in WO 2022 033985 A1, it has been confirmed for the specific triplets of antimicrobial agents proposed above that reloading and the subsequent release of the agents from 3D-printed dental appliances is generally improved if the concentration of the surfactant/emulsifier is sufficiently high. This is not particularly surprising at first, because the more emulsifier is present in the solution, the smaller the volume of the respective agent surrounded by the emulsifier in the form of a micelle, which will be beneficial for embedding the agent with the emulsifier into the 3d-printed polymer material of the appliance. Surprisingly, however, it has been found that the reloading performance drops significantly for the above triplets of essential-oil-based agents if the ratio cₐ/cₛ is below 0.05. The reason appears to be that for such low ratios of cₐ/cₛ (which is tantamount to high amounts of surfactant used in the solution of cₛ > 20 cₐ), the surfactant will be primarily embedded into the polymer material of the appliance, which is contra-productive for achieving a high respective load capacity for each agent. Above cₐ/cₛ = 0.15 (corresponding to cₛ > 6.67 cₐ), a similar drop in performance is observed, although more slowly because the emulsification becomes more and more insufficient.

Experimental results have shown that if the concentration of surfactant is too high, it will be the surfactant that is primarily embedded in the oral appliance and no longer the EOCs/the agents. This effect, of course, has a strong negative effect on the reloading performance, in particular the maximum respective reload capacity obtainable for each agent contained in the solution. The right choice of surfactant concentration is therefore crucial for obtaining efficient loading, thereby high loading capacity, therefore high release rates and ultimately efficient antimicrobial protection.

In addition, if the ratio of surfactant is too low, not all of the agents embedded into the polymer material of the appliance will be properly emulsified by the surfactant, which can be critical for the mechanical stability of the polymer-based intraoral appliance treated with the care solution, because in such a case the non-properly embedded agents can act as plasticizers thus weakening the mechanical strength of the appliance. A permanent embedding of an agent into the polymer material of the appliance should be avoided, because such an agent can no longer be released and thus cannot provide the desired antimicrobial effect; in addition, the polymer material will lose its mechanical stability over time.

The aqueous solution may also contain further ingredients, depending on desired additional effects to be achieved: For example, for whitening and remineralization of teeth, nano-hydroxyapatite (nHA) and/or calcium carbonate and/or fluoride may be contained in the solution.

To strengthen antibacterial effects, molecular iodine and/or xylit(ol) and/or erythrit may be added to the solution.

To further strengthen antibacterial effects, Glutaminyl cyclases (QC)-selective inhibitors can be added to the solution, to selectively inhibit the growth of gram-negative bacteria.

If a nutrition shall be provided with the solution as an addon, vitamins may be added.

And for rebuilding the oral flora (i.e., healthy bacteria normally found in the oral cavity), probiotics (probiotics are live microorganisms that consume prebiotics and can provide health benefits when consumed), in particular lactobacillus reuteri (e.g., from DSM 179382) and/or lactobacillus reuteri ATCC PTA 64753 may be added.

In particular a combination of prebiotics (a nutrition consumed by probiotics) and prebiotic-reducing bacteria (=probiotics) may be used as ingredients of the care solution. Suitable probiotic bacteria are in particular nitrate-reducing bacteria, preferably from the Rothia aeria bacteria strain. This approach is beneficial for improved antimicrobial efficiency: The effect which is achieved by these features is that the prebiotic and probiotic ingredients of the care solution interact in such a way that antimicrobial substances are produced in the oral cavity as a result of the metabolism of the probiotics and these substances prevent the growth of other harmful microorganisms in the oral cavity or on the dental appliance.

As mentioned before, the solution described before is ideally suited for treating intraoral appliances, in particular if based on a cellulose-based material system and/or if these appliances are 3D-printed from a polymer material (and hence show significant porosity, making them vulnerable to hazardous biofilm formation). The aqueous solutions described so far can also be used as a mouthwash or as a care solution for treating/cleaning/disinfecting oral appliances to be worn in the oral cavity and/or for cleaning and/or disinfecting surfaces (teeth and gums) in general.

Therefore, the invention also proposes solving the aforementioned task by using an aqueous solution according to the invention for loading a 3D-printed intra-oral appliance with the antimicrobial agents contained in the solution. The appliance initially loaded or reloaded with the solution can thus be an appliance that has been3D-printed from a polymer resin and/or powder material, in particular from one or more of the following polymer materials: nylon, methylacrylate based polymer, in particular polymethylmethacrylat (PMMA), ethyl methacrylate, co-polyesters, urethane (meta) acrylate oligomer, (meta) acrylate denatured siloxane resin, the di (meta) acrylate type reactive monomer, the polycarbone siloxane, and many other polymers.

In summary, a novel composition is proposed for a water-based care solution that may be employed for increasing the hygiene of intraoral appliances such as dental splints thereby improving the safety of use of intraoral appliances. By immersing such an appliance into said care solution, different active antimicrobial agents contained in the care solution can be rapidly loaded at sufficiently high amounts into the device, in particular if the device shows a highly porous surface due to an underlying 3D-printing fabrication process. Accordingly, once reloaded, the dental appliance can release the agents into the oral cavity to achieve an antimicrobial effect there and the agents remaining in the pores of the device can prevent microorganisms from growing on or inside the appliance.

## Claims

1. **Aqueous solution** with antimicrobial properties, in particular to be used
- as a mouthwash and/or
- as a care solution for treating oral appliances to be worn in the oral cavity and/or
- for cleaning and/or disinfecting surfaces and/or
- for loading/reloading active agents into a dental appliance,
**characterized in that** the solution comprises the following ingredients:
- a triplet of three different oleophilic antimicrobial essential oil components and
- a surfactant, preferably in the form of a polyglyceryl-based emulsifier.

2. Aqueous solution according to claim 1, wherein said triplet comprises methyl salicylate, cinnamaldehyde, and limonene.

3. Aqueous solution according to claim 2, wherein
limonene is replaced by / is present in the solution besides at least one of the following essential oil components: eucalyptol and/or trans-anethole and/or carvacrol and/or linalool and/or linalyl-acetate and/or eugenol and/or thymol.

4. **Aqueous solution** with antimicrobial properties, in particular according to one of the preceding claims, **characterized in that**
- the solution comprises a combination of at least two different antimicrobial agents in the form of two different oleophilic essential oil components, which show different local polarities, because at least one polar end group or side group such as
- a group featuring a carbon-oxygen double bond,
- an aldehyde (-COH) group, and/or
- an ether group (-O), and/or
- a carboxy (-COOH) group, and/or
- a methoxy group (-OCH3), and/or
- a hydroxyl group (-OH)
is present in only one of the two different essential oil components.

5. Aqueous solution according to one of the preceding claims, wherein a ratio of the total mass portion of the antimicrobial agents cₐ contained in the solution and the mass portion of the surfactant cₛ is cₐ/cₛ = [0.03 - 0.30], preferably cₐ/cₛ = [0.05 - 0.15].

6. Aqueous solution according to one of the preceding claims, wherein the solution comprises at least one prebiotic substance, in particular a nitrate-based substance.

7. Aqueous solution according to one of the preceding claims, wherein the solution comprises at least one ph-lowering agent,
- preferably at least one acidic substance,
- most preferably at least one organic acid, in particular naturally derived malic acid, tartaric acid, carboxylic acid or citric acid.

8. Aqueous solution according to one of the preceding claims, wherein the solution further comprises
- nano-hydroxyappetite (nHA) and/or
- fluoride and/or
- calcium carbonate and/or
- iodine.

9. Aqueous solution according to one of the preceding claims, wherein the solution further comprises
- xylit(hol) and/or erythrit and/or
- a vitamin.

10. Aqueous solution according to one of the preceding claims, wherein the solution further comprises
- a probiotic, in particular lactobacillus reuteri and/or Rothia bacteria species, most preferably Rothia aeria.

11. Aqueous solution according to claim 10, wherein the solution further comprises a prebiotic than can be consumed/reduced by said probiotic.

12. **Use of an aqueous solution** according to one of the claims 1 to 11,
- as a mouth wash or
- as a care solution for treating oral appliances to be worn in the oral cavity or
- for loading/reloading active agents into a dental appliance or
- for cleaning and/or disinfecting surfaces.

13. **Use of an aqueous solution** according to one of the claims 1 to 11,
- for loading a 3D-printed or thermoformed intra-oral appliance with the antimicrobial agents contained in the solution,
- in particular wherein the appliance has been 3D-printed from a polymer material, in particular from one or more of the following polymer materials: nylon, methylacrylate based polymer, in particular polymethylmethacrylat (PMMA).
